# EUROPEAN PATENT SPECIFICATION

(11) **EP 2 931 237 B1**
(45) Date of publication and mention of the grant of the patent: **04.04.2018**
(21) Application number: 13799551.0
(22) Date of filing: 04.12.2013
(51) Int. Cl.: A61K 8/46, A61Q 5/00, A61K 8/60, A61Q 5/02

(54) **PERSONAL CARE COMPOSITIONS**
KÖRPERPFLEGEZUSAMMENSETZUNG
COMPOSITION POUR SOINS PERSONNELS

(30) Priority: 17.12.2012 EP 12197527
(43) Date of publication of application: 21.10.2015
(73) Proprietor: Unilever PLC, a company registered in England and Wales under company no. 41424 of, London EC4Y 0DY (GB); Unilever N.V., 3013 AL Rotterdam (NL)
(72) Inventor: PARRY, Neil, James, Bebington Wirral Merseyside CH63 3JW (GB); STEVENSON, Paul, Simon, Bebington Wirral Merseyside CH63 3JW (GB)
(74) Representative: McHugh, Paul Edward
(86) International application number: PCT/EP2013/075475
(87) International publication number: WO 2014/095367

(56) References cited:
- WO-A1-2011/120776
- WO-A1-2012/022552
- WO-A2-2011/120780
- US-A1- 2009 156 450
- DATABASE WPI Week 201004 Thomson Scientific, London, GB; AN 2009-R39096 XP002697847, & KR 2009 0117081 A (LG HOUSEHOLD&HEALTHCARE LTD) 12 November 2009 (2009-11-12)
- DATABASE WPI Week 201118 Thomson Scientific, London, GB; AN 2011-B53707 XP002697848, & JP 2011 026277 A (TOYOBO KK) 10 February 2011 (2011-02-10)
- DATABASE WPI Week 201120 Thomson Scientific, London, GB; AN 2011-C30435 XP002697849, & JP 2011 046634 A (TOYOBO KK) 10 March 2011 (2011-03-10)

## Description

The invention relates to a personal care compositions comprising biosurfactants as co-surfactants.

Biosurfactants offer a wide range of advantages as co-surfactants, not least their environmental advantages. However, large scale transfer from petrochemical surfactants to biosurfactants in composition remains a challenge. One problem arises from the effect of biosurfactants on certain key performance characteristics in so far as consumer interaction is concerned. As a result of these effects, biosurfactants remain under-utilised in mass consumer compositions and there is continued reliance of the industry on petrochemical surfactants for the major proportion of the surfactant in the final product.

It is an object of the invention to provide an improved personal care compositions. The invention provides a personal care composition comprising:-
a) a surfactant combination comprising (i) a synthetic anionic surfactant; and (ii) a biosurfactant comprising a glycolipid wherein the biosurfactant is present at a level from wt.50 % to 75% of the total surfactant in said surfactant combination
b) a fatty acyl isethionate product at a level of 4-5% wt.% of the total personal care composition.

With the invention, biosurfactants can be incorporated at high levels whilst desired and advanced rheology profiles can be improved and even restored to the level attained with synthetic surfactants alone. The invention allows accurate control of rheology profiles for personal care compositions.

As used herein "wt %" means weight percent. All percentages given are by weight unless specified and are percentages of the total composition unless specified. Compositions of the invention may be used in either a personal wash liquid cleansing base or in cosmetic composition base.

The compositions of the invention comprise pourable liquids and preferably have a viscosity in the range 250 to 100,000 mPas (cP) measured at a shear rate 10s@-1 and 25 DEG C., in a Haake Rotoviscometer RV20. Shampoo compositions are preferably in the range 5000-8000 cP.

The composition according to the invention can take the form of a liquid, intended to be dispensed from a capped container such as a bottle, roll-on applicator or tube, or a pump-operated dispenser and may be a skin cleanser, shower product, bath additive or shampoo.

Compositions of the invention may be formulated as products for washing the skin, for example, bath or shower gels, hand washing compositions or facial washing liquids; pre- and post-shaving products; rinse-off, wipe-off and leave-on skin care products; products for washing the hair and for dental use.

### Biosurfactants

The biosurfactant preferably comprises a microbially-derived biosurfactant. Preferably it comprises a glycolipid biosurfactant moiety which may be a rhamnolipid or sophorolipid or trehalolipid or a mannosylerythritol lipid (MEL) or combinations thereof.

Alternatively or additionally the biosurfactant may comprise any shear thinning biosurfactant and in this respect, may extend to include any shear thinning glycolipid biosurfactant mentioned above or any shear thinning cellobiose , peptide based biosurfactant, lipoprotein, lipopeptide e.g. surfactin, fatty acids e.g. corynomucolic acids (preferably with hydrocarbon chain C12-C14), phospholipid (e.g. Phosphatidylethanolamine produced by *Rhodococcus erythropolis* grown on n-alkane resulted in the lowering of interfacial tension between water and hexadecane to less than 1 mN m⁻¹ and CMC of 30 mg L⁻¹ (Kretschner *et al.,* 1982)), spiculisporic acid, polymeric biosurfactants including emulsan, liposan, mannoprotein or polysaccharide-protein complexes or combinations thereof.

Preferably the biosurfactant moiety comprises a rhamnolipid.

The biosurfactant moiety may comprise one or more saccharide moieties such as sugar rings. In the case of rhamnolipids the rhamnolipid may comprise one or both components: mono-rhamnolipids having a single rhamnose sugar ring and di-rhamnolipids, having two rhamnose sugar rings.

In the case of rhamnolipids, throughout this patent specification, the prefixes mono- and di- are used to indicate respectively to indicate mono-rhamnolipids (having a single rhamnose sugar ring) and di-rhamnolipids (having two rhamnose sugar rings) respectively. If abbreviations are used R1 is mono-rhamnolipid and R2 is di-rhamnolipid.

The biosurfactant can be used to replace at least 50 wt.% of the synthetic anionic surfactant but with the rheology profile tuned to match that of the original synthetic surfactant using the fatty isethionate as a rheology modifier. The biosurfactant is present at a level 50- 75% wt.% of the surfactant.

### Fatty acyl isethionate product

The fatty acyl isethionate product is present at a level of from 4 to 5 wt.% (of the total composition).

The preferred fatty acyl isethionate product comprises fatty acyl isethionate t at a level of from 40 to 80 wt.% of the product, as well as free fatty acid and/or fatty acid salt at a level of from 15 to 50 wt.%.

Preferably, greater than 20 wt.% and less than 45 wt. %, more preferably greater than 25 wt.% and less than 45 wt. % of the fatty acyl isethionate are of chain length greater than or equal to C₁₆; and greater than 50 wt.%, preferably greater than 60 wt.% of the free fatty acid/soap is of chain length C₁₆ to C₂₀.

The fatty acyl isethionate surfactant component is typically prepared by the reaction of an isethionates salt such as alkali metal isethionates and an aliphatic fatty acid having 8 to 20 carbon atoms and Iodine Value (measuring degree of unsaturation) of less than 20 g, for example:

RCOOH + HOR₁SO₃M → RCOOR₁SO₃M

where R₁ is an aliphatic hydrocarbon radical containing 2 to 4 carbons;
M is alkali metal cation or metal ion (e.g., sodium, magnesium, potassium, lithium), ammonium or substituted ammonium cation or other counterion; and,
R is an aliphatic hydrocarbon radical having 7 to 24, preferably 8 to 22 carbons.

Depending on the processing conditions used, the resulting fatty acyl isethionate product can be a mixture of 40 to 80% by weight of fatty acyl isethionates (which formed from the reaction) and 50 to about 15 wt. %, typically 40 to 20 wt. % of free fatty acids. In addition, the product may contain isethionates salts which are present typically at levels less than 5 wt. %, and traces (less than 2 wt. %) of other impurities. Preferably, a mixture of aliphatic fatty acids is used for the preparation of commercial fatty acyl isethionates surfactants. The resulting fatty acyl isethionate surfactants (e.g., resulting from reaction of alkali metal isethionate and aliphatic fatty acid) preferably should have more than 20 wt. %, preferably more than 25 wt.%, but no more than 45 wt.%, preferably 35 wt. % (on basis of fatty acyl isethionates reaction product) of fatty acyl group with 16 or greater carbon atoms to provide both excellent lather and mildness of the resulting fatty acyl isethionate product. These longer chain fatty acyl isethionate surfactants and fatty acids, i.e. fatty acyl group and fatty acid with 16 or more carbons, can typically form insoluble surfactant/fatty acid crystals in water at ambient temperatures.

Examples of commercial fatty acyl isethionate products that are particularly useful in the subject invention are DEFI flakes and Dove® cleansing bar noodles produced by Unilever. DEFI (Direct Esterification of Fatty Isethionate) flakes typically contain about 68 to 80 wt. % of sodium fatty acyl isethionate and 15 to 30 wt. % free fatty acid. More than 25 wt. % and no more than 35 wt.% of fatty acyl group of the resulting fatty acyl isethionate have 16 to 18 carbon atoms. Dove® cleansing bar noodles are mixtures of DEFI flakes described above and long chain (mainly C₁₆ and C₁₈) fatty acid and fatty soap which contain about 40 to 55 wt. % of fatty acyl isethionate and 30 to 40 wt. % of fatty acid and fatty soap.

### Further Surfactants

Total surfactant in personal wash compositions, for example, will generally comprise 5 to 60% by wt, preferably 10 to 40% by wt surfactant, preferably 2-16 % by wt while cosmetic compositions preferably comprise 1% to 30% by wt., more preferably 1 to 15% by wt. surfactant.

The total amount of surfactant in shampoo compositions of the invention may generally range from 0.5 to 45 wt.%, preferably from 1.5 to 35 wt.%, more preferably from 5 to 20 wt.%, calculated as a weight percentage total weight of the composition.

The surfactant combination of the invention may provide the total surfactant component of the composition, or further surfactants may be included, in addition to the surfactant combination.

The further surfactant which may include anionic surfactants as well as nonionic, amphoteric and zwitterionic surfactants depending on product form.

Preferred personal wash compositions comprise mixtures of anionic and amphoteric surfactants.

The compositions according to the invention can optionally comprise, as a surfactant one or more soaps which are water-soluble or water-dispensable alkali metal salts of an organic acid, especially a sodium or a potassium salt, or the corresponding ammonium or substituted ammonium salt. Examples of suitable organic acids are natural or synthetic alkanoic acids having from 10 to 22 carbon atoms, especially the fatty acids of triglyceride oils such as tallow and coconut oil. For solid products, such as powders, bars or tablets, the preferred soap is a soap of tallow fatty acids. Minor amounts of up to about 30% by weight, preferably 10 to 20% by weight of sodium soaps of nut oil fatty acids derived from nut oils, for example coconut oil and palm kernel oil, may be admixed with the sodium tallow soaps, to improve their lathering and solubility characteristics if desired.

The preferred soap are predominantly C10-C14 fatty acids derived from nut oils, or alternatively, from synthetic alkanoic acids.

The soaps can be provided as a performed ingredient for the composition, or they can be formed *in situ* during the manufacture of the composition by reaction of suitable fatty acids and an alkali.

The amount of fatty acid soap which can be present in the composition according to the invention is up to 90% by weight, preferably from 2-80% by weight of the composition.

The composition according to the invention can also optionally comprise one or more non-soap anionic surfactants, examples of which include:
The alkali metal salts of organic sulfuric reaction products having an alkyl or acyl radical containing from 8-22 carbon atoms and a sulphonic acid or sulfuric acid ester group. Specific examples of these synthetic anionic surfactants are the sodium, ammonium, potassium or triethanolammonium alkyl sulphates, especially those obtained by sulphating the higher alcohols (C8 -C18), sodium coconut oil fatty acid monoglyceride sulphates and sulphonates; sodium or potassium salts of sulfuric esters of the reaction product of one mole of a higher fatty alcohol (e.g., tallow or coconut oil alcohols) and 1-12 moles of ethylene oxide; sodium or potassium salts of alkylphenol ethylene oxide ether sulphate with 1-10 units of ethylene oxide per molecule and in which the alkyl group contains from 8 to 12 carbon atoms, sodium alkyl glyceryl ether sulphonates, the reaction product of fatty acids having from 10 to 22 carbon atoms esterified with isethionic acid and neutralized with sodium hydroxide; water soluble salts of condensation products of fatty acids with N-methyl taurine. Especially preferred non-soap anionic surfactants include:
   alkylaryl sulphonates, such as sodium alkyl benzene sulphonate (e.g., TEEPOL CM44, available from Shell), alkyl sulphates, such as sodium lauryl sulphate (e.g., EMPICOL CX, available from Albright & Wilson), and triethanolamine lauryl sulphate (e.g., EMPICOL TL40/T, available from Albright & Wilson);
   alkyl ether sulphates, such as sodium lauryl ether sulphate (e.g., EMPICOL ESB70, available from Albright & Wilson);
   alkyl sulphonates, such as sodium alkane (C13-18) sulphonate (e.g., HOSTAPUR SAS 30, available from Hoechst);
   olefin sulphonates, such as sodium olefin sulphonate (C15-18) (e.g., HOSTAPUR OS, available from Hoechst);

Sarcosinates, having the structure (3): 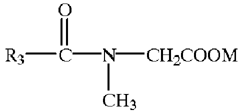 where
R₃ is chosen from C6-14 alkyl, and M is a counterion chosen from alkali metals, ammonium, substituted ammonium, such as alkanolammonium.

An example of sarcosinates having the structure (3) sodium lauryl sarcosinate (e.g., HAMPOSYL L-95, available from Grace).

Taurides, having the structure (4): 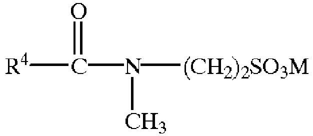 where
R⁴ is chosen from C8-18 alkyl.

An example of taurides having the structure (4) is: coconut methyl taurine (e.g., FENOPON TC 42, available from GAF).

Isethionates, having the structure (5): 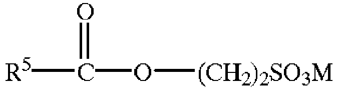 where:
R⁵ is chosen from C8-18 alkyl.

An example of isethionates having the structure (5) is: sodium acyl isethionate (e.g., JORDAPON CI, available from Jordan).

Monoalkyl sulphosuccinates, having the structure (6): 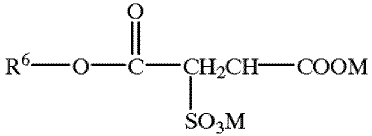 where
R⁶ is chosen from C10-20 alkyl.

Examples of monoalkyl sulphosuccinates having this structure (6) include: sodium lauryl sulphosuccinate (e.g., EMPICOL SLL, available from Albright & Wilson); magnesium alkyl sulphosuccinate (e.g., ELFANOL 616 Mg. available from AKZO), sodium lauryl ethoxysulphosuccinate (e.g., EMPICOL SDD, available from Albright & Wilson), coconut monoethanolamide ethoxysulphosuccinate, (e.g., EMPICOL SGG); disodium lauryl polyglycol ether sulphosuccinate (e.g., SURTAGENE S30, available from CHEM-Y) polyethyleneglycol sulphosuccinate (e.g., REWOPOL SBFA 30, available from REWO). Dialkyl sulphosuccinates, having the structure (7): 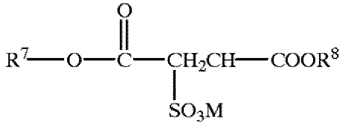 where
R⁷ and R⁸ are the same or different, and are chosen from C6-14 alkyl.

An example of dialkyl sulphosuccinate having the structure (7) is: sodium dioctyl sulphosuccinate (e.g. EMCOL 4500 available from Witco).

Acyl lactylates, having the structure (8): 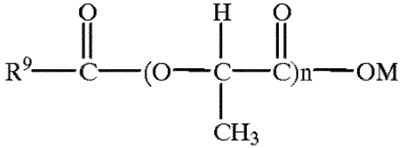 where
R⁹ is chosen from C6-16 alkyl.

An example of acyl lactylates having the structure (8) is: decanoyl lactylate (e.g., PATIONIC 122A, available from Patterson, C. J.).

Acylated .alpha.-amino acids, such as sodium lauryoyl glutamate (e.g., ACYL GLUTAMATE LS-11, available from Ajinomoto Co. Inc.).

Ethyl carboxylates, such as alkyl C12-14 O(EO)4 OCH--2 CO2 Na (e.g., AKYPO RLM 38, available from AKZO).

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. A general formula for these compounds is: 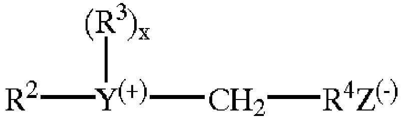 wherein R² contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; R³ is an alkyl or monohydroxyalkyl group containing about 1 to about 3 carbon atoms; X is 1 when Y is a sulfur atom, and 2 when Y is a nitrogen or phosphorus atom; R4 is an alkylene or hydroxyalkylene of from about 1 to about 4 carbon atoms and Z is a radical selected from the group consisting of carboxylate, sulfonate, sulfate, phosphonate, and phosphate groups.

Examples of such surfactants include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxypropane-1-pho sphate;
3-[N,N-dipropyl-N-3-dodecoxy-2-hydroxypropylammonio]-propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxyethyl)-N-(2-hydroxydodecyl)ammonio]-butane-1-carboxylate;
3-[S-ethyl-S-(3-dodecoxy-2-hydroxypropyl)sulfonio]-propane-1-phosphate;
3-[P,P-dimethyl-P-dodecylphosphonio]-propane-1-phosphonate; and
5-[N,N-di(3-hydroxypropyl)-N-hexadecylammonio]-2-hydroxy-pentane-1-sulfate.

Amphoteric detergents which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 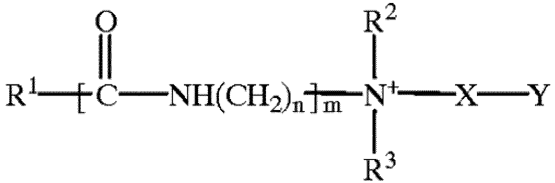 where
R¹ is alkyl or alkenyl of 7 to 18 carbon atoms;
R² and R³ are each independently alkyl, hydroxyalkyl or carboxyalkyl of 1 to 3 carbon atoms;
n is 2 to 4;
m is 0 to 1;
X is alkylene of 1 to 3 carbon atoms optionally substituted with hydroxyl, and
Y is -CO2- or -SO3-

Suitable amphoteric detergents within the above general formula include simple betaines of formula: 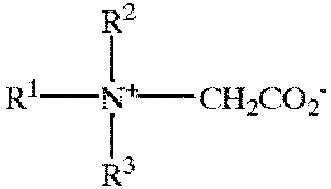 and amido betaines of formula: 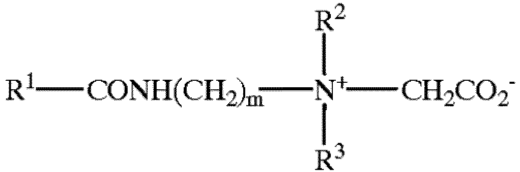 where m is 2 or 3.

In both formulae R¹, R² and R³ are as defined previously. R¹ may in particular be a mixture of C12 and C14 alkyl groups derived from coconut so that at least half, preferably at least three quarters of the groups R¹ have 10 to 14 carbon atoms. R² and R³ are preferably methyl.

A further possibility is that the amphoteric detergent is a sulphobetaine of formula : 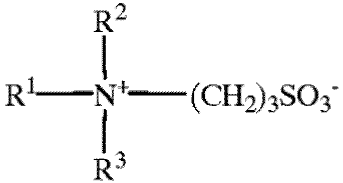 or 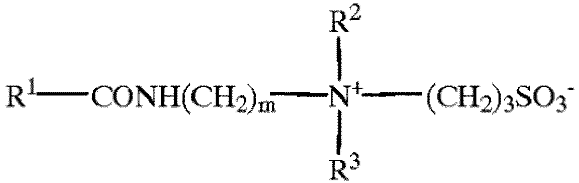 where m is 2 or 3, or variants of these in which -(CH₂)₃SO⁻₃ is replaced by:

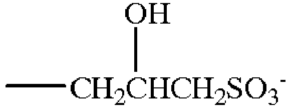

In these formulae R¹, R² and R³ are as discussed previously.

Amphoacetates and diamphoacetates are also intended to be covered in possible zwitterionic and/or amphoteric compounds which may be used. Amphoteric/zwitterionic, when used, generally comprises 0 to 25%, preferably 0.1 to 20% by wt. of the composition.

The surfactant system may optionally comprise a nonionic surfactant.

The nonionic which may be used includes in particular the reaction products of compounds having a hydrophobic group and a reactive hydrogen atom, for example aliphatic alcohols, acids, amides or alkyl phenols with alkylene oxides, especially ethylene oxide either alone or with propylene oxide. Specific nonionic detergent compounds are alkyl (C6 -C22) phenols-ethylene oxide condensates, the condensation products of aliphatic (C8 -C18) primary or secondary linear or branched alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxides.

The nonionic may also be a sugar amide, such as a polysaccharide amide. Specifically, the surfactant may be one of the lactobionamides described in U.S. Pat. No. 5,389,279 to Au et al. which is hereby incorporated by reference or it may be one of the sugar amides described in U.S. Pat. No. 5,009,814 to Kelkenberg, hereby incorporated into the subject application by reference.

Preferred alkyl polysaccharides are alkylpolyglycosides of the formula

R²O(CₙH₂ₙO)ₜ (glycosyl)ₓ

wherein R² is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

Nonionic surfactant typically comprises 0 to 10% by wt. of the composition.

If present in liquid personal wash composition, the surfactant system of the invention may comprise 5% to 60% by wt., preferably 10-40% by wt. of a surfactant system which preferably comprises:
(a) 1% to 20% by wt. one or more anionics (surfactant mix) as described above;
(b) 0.1 to 20% by wt. amphoteric/zwitterionic;
(c) 0 to 10% by wt. nonionic surfactant.

Anionics, amphoteric/zwitterionic and nonionics are as described above. A preferred anionic-amphoteric surfactant combination is acyl isethionate and amphoteric is betaine such as cocoamidoalkylbetaine.

Personal wash compositions according to the invention may optionally also include structurant. Suitable structuring materials include swelling clays, for example laponite; fatty acid and derivatives thereof, in particular, fatty acid monoglyceride polyglycol ethers; cross-linked polyacrylates such as Carbopol (TM) (polymers available from Goodrich); acrylates and copolymers thereof; polyvinylpyrrolidone and copolymers thereof; polyethyleneimines; salts such as sodium chloride and ammonium sulphate; sucrose esters; gellants; and mixtures thereof.

Of the clays, particularly preferred are synthetic hectorite (laponite) clay used in conjunction with an electrolyte salt capable of causing the clay to thicken. Suitable electrolytes include alkali and alkaline earth salts such as halides, ammonium salts and sulphates.

The composition may also comprise internal lamellar phase-inducing structurants. Such structurants include C8-C24 unsaturated and/or branched liquid fatty acid or esters thereof; C8-C24 unsaturated and/or branched liquid alcohol or ether thereof; and/or C5 to C9 fatty acids wherein those structuring have MP below 25 DEG C.

When present, structurants may comprise 0.1 to 25% by weight, preferably 1 to 15% weight of composition.

The personal wash formulations may comprise a thickening (or thinning) agent, i.e., a material which assists the rheology of the composition, by increasing or decreasing the viscosity of this phase as the shear rate thereof is increased during use. Examples of such agents are include cross-linked polyacrylates such as Carbopol (TM) (polymers available from Goodrich); natural gums including alginates, guar, xanthan and polysaccharide derivatives including carboxy methyl cellulose and hydroxypropyl guar; propylene glycols and propylene glycol oleates; salts such as sodium chloride and the ammonium sulphate; glycerol tallowates; and mixtures thereof.

These agents may comprise 1% to 15% by wt. of the composition.

Other optional components of such compositions include opacifiers, preferably 0.2 to 2.0 wt. %; preservatives, preferably 0.2 to 2.0 wt. %; and perfumes, preferably 0.5 to 2.0 wt. %. Cationic polymers such as Jaguar.RTM. from Rhone Poulenc and Polymer JR.RTM. from Amerchol may also be included.

The base compositions may further comprise additional oil/emollient particles (particularly when in lamellar phase) wherein the additional benefit agent (i.e., in addition to the .alpha.-hydroxy acid mixture benefit agent composition) may be as set forth below:
Vegetable oils: Arachis oil, cannola oil, castor oil, cocoa butter, coconut oil, corn oil, cotton seed oil, olive oil, palm kernel oil, rapeseed oil, safflower seed oil, sesame seed oil and soybean oil.

Esters: Butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate.

Animal Fats: Acytylatelte lanolin alcohols, lanolin, lard, mink oil and tallow.

Fatty acids and alcohols: Behenic acid, palmitic acid, stearic acid, behenyl alcohol, cetyl alcohol, eicosanyl alcohol and isocetyl alcohol.

Other examples of oil/emollients include mineral oil, petrolatum, silicone oil such as dimethyl polysiloxane, lauryl and myristyl lactate.

Additional emollient/oil generally will comprise, if present, 1% to 20% by weight of the composition.

Other ingredients which may be found in such personal care compositions are as follows:
Organic solvents, such as ethanol; auxiliary thickeners, such as carboxymethylcellulose, magnesium aluminum silicate, hydroxyethylcellulose, methylcellulose, carbopols, glucamides, or Antil.RTM. from Rhone Poulenc; perfumes; sequestering agents, such as tetrasodium ethylenediaminetetraacetate (EDTA), EHDP or mixtures in an amount of 0.01 to 1%, preferably 0.01 to 0.05%; and coloring agents, opacifiers and pearlizers such as zinc stearate, magnesium stearate, TiO2, EGMS (ethylene glycol monostearate) or Lytron 621 (Styrene/Acrylate copolymer); all of which are useful in enhancing the appearance or cosmetic properties of the product.

The compositions may further comprise antimicrobials such as 2-hydroxy-4,2'4'trichlorodiphenylether (DP300); preservatives such as dimethyloldimethylhydantoin (Glydant XL1000), parabens, sorbic acid etc.

The compositions may also comprise coconut acyl mono- or diethanol amides as suds boosters, and strongly ionizing salts such as sodium chloride and sodium sulfate may also be used to advantage.

Antioxidants such as, for example, butylated hydroxytoluene (BHT) may be used advantageously in amounts of about 0.01% or higher if appropriate.

Cationic conditioners which may be used include Quatrisoft LM-200 Polyquaternium-24, Merquat Plus 3330--Polyquaternium 39; and Jaguar.RTM. type conditioners.

Polyethylene glycols which may be used include: Polyox WSR-205 PEG 14M, Polyox WSR-N-60K PEG 45M, or Polyox WSR-N-750 PEG 7M.

Other thickeners which may be used include Amerchol Polymer HM 1500 (Nonoxynyl Hydroethyl Cellulose); Glucam DOE 120 (PEG 120 Methyl Glucose Dioleate); Rewoderm.RTM. (PEG modified glyceryl cocoate, palmate or tallowate) from Rewo Chemicals; Antil.RTM. 141 (from Goldschmidt).

Another optional ingredient which may be added are the defloculating polymers such as are taught in U.S. Pat. No. 5,147,576 to Montague, hereby incorporated by reference.

Another ingredient which may be included are exfoliants such as polyoxyethylene beads, walnut sheets and apricot seeds.

If present as a cosmetic "leave-on" composition, the compositions generally will contain less surfactant preferably 1-15% by wt. but include more ingredients characteristic of cosmetic or commercially acceptable vehicle.

The cosmetic vehicle composition (comprising 1% to 99% by weight of total cosmetic, preferably 1-80% by weight of total cosmetic) may comprise an oil or oily material, together with an emulsifier, to provide either a water-in-oil emulsion or an oil-in-water emulsion, depending largely on the average hydrophilic-lipophilic balance (HLB) of the emulsifier employed.

Various types of active ingredients may be present in cosmetic vehicle compositions of the present invention. Actives are defined as skin or hair benefit agents other than emollients and other than ingredients that merely improve the physical characteristics of the composition. Although not limited to this category, general examples include sunscreens, tanning agents.

Sunscreens include those materials commonly employed to block ultraviolet light. Illustrative compounds are the derivatives of PABA, cinnamate and salicylate. For example, octyl methoxycinnamate and 2-hydroxy-r-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively. The exact amount of sunscreen employed in the emulsions can vary depending upon the degree of protection desired from the sun's UV radiation.

Typically actives will comprise 1% to 30% by weight of the total cosmetic composition.

Another preferred optional ingredient is selected from essential fatty acids (EFAs), i.e., those fatty acids which are essential for the plasma membrane formation of all cells, in keratinocytes EFA deficiency makes cells hyperproliferative. Supplementation of EFA corrects this. EFAs also enhance lipid biosynthesis of epidermis and provide lipids for the barrier formation of the epidermis. The essential fatty acids are preferably chosen from linoleic acid, .gamma.-linolenic acid, homo-.gamma.-linolenic acid, columbine acid, eicosa-(n-6, 9, 13)-trienoic acid, arachidonic acid, .gamma.-linolenic acid, timnodonic acid, hexaenoic acid and mixtures thereof.

Emollients are often incorporated into cosmetic compositions of the present invention. Levels of such emollients may range from about 0.5% to about 50%, by weight preferably between about 5% and 30% by weight of the total cosmetic composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate(a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention are thickeners. A thickener will usually be present in amounts anywhere from 0.1 to 20% by weight, preferably from about 0.5% to 10% by weight of the total composition. Exemplary thickeners are cross-linked polyacrylate materials available under the trademark Carbopol from the B. F. Goodrich Company. Nonionic cellulose materials such as methyl cellulose and hydroxy propyl methyl and cellulose may be used. Also cationic cellulose materials such as polymer JR400 and cationic gums such as Jaguar <135 may be used as thickeners.

Surfactants, which are also sometimes designated as emulsifiers, may be incorporated into the cosmetic compositions of the present invention. Surfactants can comprise anywhere from about 0.5 to about 30% by weight, preferably from about 1 to about 15% by weight of the total composition. Surfactants may be cationic, nonionic, anionic, or amphoteric in nature and combinations thereof may be employed and examples of various surfactants are described above. In this respect the surfactant combination of the invention may comprise surfactants having emulsifying benefits.

The composition according to the invention can also contain other optional adjuncts, that is ingredients other than the main ingredients already defined which are conventionally employed in compositions for topical application to human skin. These adjuncts, when present, will normally form the balance of the composition.

Examples of optional adjuncts include vehicles, the selection of which will depend on the required product form of the composition. Typically, the vehicle when present, will be chosen from diluents, dispersants or carriers for the ingredients so as to ensure an even distribution of it when applied to the skin.

The compositions may include water as a vehicle in combination with at least one other cosmetically-acceptable vehicle.

Vehicles other than water that can be used in compositions according to the invention can include liquids or solids as emollients, solvents, humectants, thickeners and powders. Examples of each of these types of vehicles, which can be used singly or as mixtures of one or more vehicles, are as follows:
Emollients, such as stearyl alcohol, glyceryl monolaurate, glyceryl monoricinoleate, glyceryl monostearate, propane-1,2-diol, butane-1,3-diol, docosan-1,2-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, soybean oil, sunflower seed oil, olive oil, sesame seed oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

Propellants, such as trichlorofluoromethane, dichlorodifluoromethane, dichlorotetrafluoroethane, monochlorodifluoromethane, trichlorotrifluoroethane, propane, butane, isobutane, dimethyl ether, carbon dioxide, nitrous oxide;

Solvents, such as ethyl alcohol, methylene chloride, isopropanol, acetone, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicondioxide, sodium polyacrylate, tetra alkyl and/or trialkyl aryl ammonium smectites, chemically modified magnesium aluminum silicate, organically modified montmorillonite clay, hydrate aluminum silicate, fumed silica, carboxyvinyl polymer, sodium carboxymethyl cellulose, ethylene glycol monostearate.

The cosmetically acceptable vehicle, when present, will usually form from 0.01 to 99.9% by weight, preferably from 50 to 98% by weight of the composition, and can, in the absence of other cosmetic adjuncts, form the balance of the composition.

A typical cosmetic composition will comprise:
(a) 0.05 to 30 wt.%, preferably 1 to 15 wt.% surfactant combination as described herein;
(b) 1 to 98 wt.% of a composition which composition comprises of
   (i) 1 to 20wt.% total cosmetic composition of actives;
   (ii) 1 to 15 wt.% total composition essential fatty acids;
   (iii) 0.5 to 50 wt,% total composition emollient; and
   (iv) 0.1 to 20wt.% total rheology modifier comprising a fatty acyl isethionate product as described herein.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The Examples will now be illustrated with reference to the following non-limiting Examples. Inventions according to the invention are demonstrated by a number, comparative inventions are demonstrated by a letter.

### Examples

| **_Ingredient name** | **Tradename** | **Ex.1 wt.%** | **Ex.2 wt.%** | **Ex.3 wt.%** | **Ex. 4 wt. %** |
|---|---|---|---|---|---|
| SLES1 EO is ethoxylated alkyl sulfate anionic surfactant having an average degree of ethoxylation of 1. | Texapon N701 | 12 | 6 | 6 | 6 |
| BIOSURFACTANT ⁴ | JBR425 or SL18 as indicated in results | 0 | 3 | 3 | 3 |
| Cocamidopropyl Betaine | Tegobetaine CK | 1.6 | | | |
| Fatty Acyl Isethionate Product¹ | | 0 | 0 | 2 | 4 |
| Carbo mer | Carbopol 980 | 0.4 | | | |
| Silicone Oil² | | 2.2 | | | |
| Guar Hydroxypropyltrimonium Chloride | Jaguar C14S | 0.2 | | | |
| Parfum | | 0.7 | | | |
| Aqua + minors | Water + minors | to 100 | | | |

| | | | | | |
|---|---|---|---|---|---|
| ¹The Fatty Acyl Isethionate product is Sodium Cocoyl isethionate, Stearic Acid, Coconut Fatty Acid, Sodium Isethionate and Water produced in-house by Unilever ² Mixture of silicone emulsions from Wacker and Dow ³ The Viscosity of the formulations was measured using a Brookfield viscometer at 30°C and 20rpm using spindle N5; all were in the range of from 5000 to 8000 Cp ⁴ The biosurfactant is JBR425 (CAS no. 147858-26-2) ex. Jeneil Biosurfactant Co., LLC OR is SL18 ex Ecover Belgium N.V. - Industrieweg 3, B-2890 Malle, Belgium; as indicated in the results. * The results of varying the sophorolipid biosurfactant and the fatty acyl isethionate levels and biosurfactant levels are shown in figure 1 wherein: | | | | | |

Diamond point curve- is the rheology profile of a shampoo formulation according to Example 1 with 12% SLES 1EO as a reference shampoo Square point curve - 50% replacement of SLES 1EO by SL 18 - viscosity is reduced by a factor 8

Triangle point curve - 50% replacement of SLES 1EO by SL 18 + 2% fatty acyl isethionate product¹ - increase of the viscosity compare to formulation without fatty acyl isethionate product¹ but still not matching the reference viscosity Circle point curve - 50% replacement of SLES 1 EO by SL 18 + 4% fatty acyl isethionate product¹ - viscosity in now matching the reference especially in term of pouring

| **Formulation name** | **Viscosity at 1s-1 (PaS)** | **Viscosity at 21s-1 (PaS)** | **Viscosity at106s-1 (PaS)** |
|---|---|---|---|
| **Shampoo 100%SLES1 EO** | 3.834 | 2.105 | 1.069 |
| **Sophorolipd SL18 50%** | 0.454 | 0.243 | 0.173 |
| **Sophorolipd SL 18 50%** - **2%** fatty acyl isethionate product¹ | 0.711 | 0.372 | 0.263 |
| **Sophorolipid SL18 50% - 4%** fatty acyl isethionate product¹ | 4.130 | 2.407 | 1.234 |

| | | | |
|---|---|---|---|
| * The results of varying the rhamnolipd biosurfactant and the fatty acyl isethionate product¹ levels and biosurfactant levels are shown in figure 2 wherein: | | | |

Diamond point curve- is the rheology profile of a shampoo formulation according to Example 1 with X% SLES 1EO as a reference shampoo

Square point curve - 50% replacement of SLES 1EO by JBR 425 - viscosity is reduce by a factor 2

Triangle point curve - 50% replacement of SLES 1EO by JBR 425 + 2% fatty acyl isethionate product¹ - No change in the trend compare to the JBR 425 alone Circle point curve - 50% replacement of SLES 1EO by JBR 425 + 4% fatty acyl isethionate product¹ - viscosity increased toward reference profile

| **Formulation name** | **Viscosity at 1s-1 (PaS)** | **Viscosity at 21s-1 (PaS)** | **Viscosity at106s-1 (PaS)** |
|---|---|---|---|
| **Shampoo 100%SLES1EO** | 3.8336 | 2.1049 | 1.0688 |
| **JBR425 50% - 0%** | 1.8327 | 0.7921 | 0.5224 |
| **JBR425 50% - 2%** fatty acyl isethionate product¹ | 1.4915 | 0.8187 | 0.6105 |
| **JBR425 50% - 4%** fatty acyl isethionate product¹**I** | 2.2157 | 1.2804 | 0.9908 |

The data clearly shows that the inclusion of a fatty acyl isethionate product as defined herein provides a shampoo having improved rheological properties.

### Viscosity Measurement:

The Viscosity of these shampoo formulations was measured using a Brookfield viscometer at 30°C and 20rpm using spindle N5.

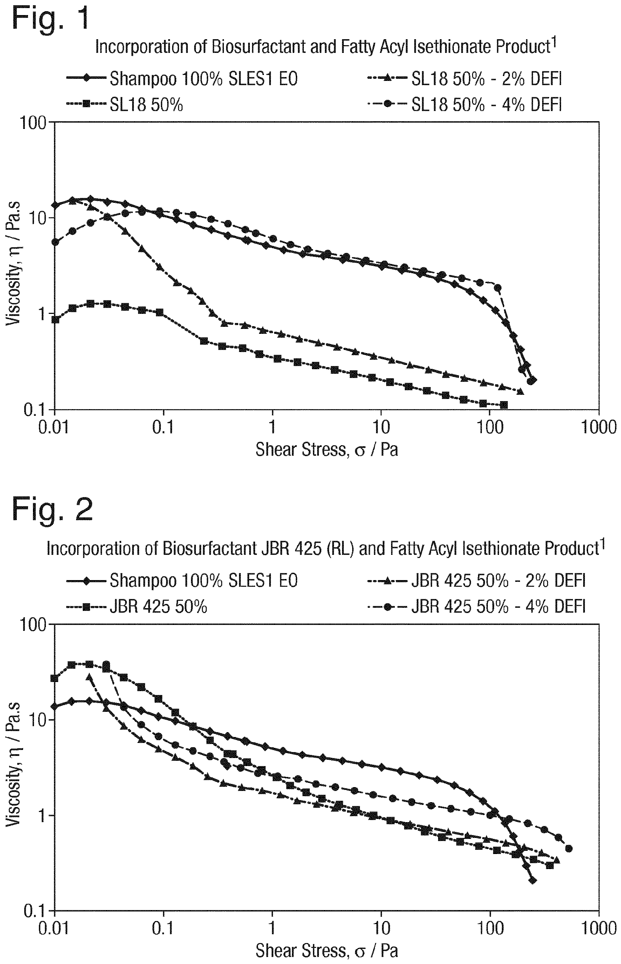

## Claims

1. A personal care composition comprising:-
a) a surfactant comprising (i) a synthetic anionic surfactant; and (ii) a biosurfactant comprising a glycolipid wherein the biosurfactant is present at a level of from 50 wt.% to 75 wt.% of the total surfactant combination; and
b) a fatty acyl isethionate product at a level of 4 wt.% to 5 wt.% of the total personal care composition.

2. A personal care composition according to any preceding claim wherein the biosurfactant comprises a rhamnolipid or sophorolipid or trehalolipid or a mannosylerythritol lipid (MEL) or combinations thereof.

3. A personal care composition according to any preceding claim wherein the biosurfactant comprises a rhamnolipid.

4. A personal care composition according to any preceding claim wherein the biosurfactant comprises a sophorolipid.

5. A personal care composition according to any preceding claim wherein the biosurfactant comprises one or more saccharide moieties.

6. A personal care composition according to any preceding claim wherein the fatty acyl isethionate product comprises fatty acyl isethionate at a level of from 40 to 80 wt.% of the product, as well as free fatty acid and/or fatty acid salt at a level of from 15 to 50 wt.%.

7. A personal care composition according to any preceding claim wherein the fatty acyl isethionate product comprises greater than 20 wt.% and less than 45 wt. %, more preferably greater than 25 wt.% and less than 45 wt. % of chain length greater than or equal to C₁₆; and greater than 50 wt.%, preferably greater than 60 wt.% of the free fatty acid/soap is of chain length C₁₆ to C₂₀.

8. A personal care composition according to any preceding claim wherein the composition is a personal wash composition and the surfactant combination comprises 5 to 60% by wt.

9. A personal care composition according to any of claims 1-7 wherein the composition is a cosmetic composition and comprises 1 to 30% by wt. of surfactant combination.

## Patentansprüche

1. Körperpflegezusammensetzung, umfassend:
a) ein Tensid, umfassend (i) ein synthetisches anionisches Tensid und (ii) ein Biotensid, das ein Glycolipid umfasst, wobei das Biotensid in einer Konzentration von 50 Gew.-% bis 75 Gew.-% der gesamten Tensidkombination vorliegt, und
b) ein Fettacylisethionat-Produkt in einer Konzentration von 4 Gew.-% bis 5 Gew.-% der gesamten Körperpflegezusammensetzung.

2. Körperpflegezusammensetzung nach einem vorhergehenden Anspruch, wobei das Biotensid ein Rhamnolipid oder Sophorolipid oder Trehalolipid oder ein Mannosylerythritollipid (MEL) oder Kombinationen davon umfasst.

3. Körperpflegezusammensetzung nach einem vorhergehenden Anspruch, wobei das Biotensid ein Rhamnolipid umfasst.

4. Körperpflegezusammensetzung nach einem vorhergehenden Anspruch, wobei das Biotensid ein Sophorolipid umfasst.

5. Körperpflegezusammensetzung nach einem vorhergehenden Anspruch, wobei das Biotensid ein oder mehrere Saccharid-Reste umfasst.

6. Körperpflegezusammensetzung nach einem vorhergehenden Anspruch, wobei das Fettacylisethionat-Produkt Fettacylisethionat in einer Konzentration von 40 bis 80 Gew.-% des Produktes sowie freie Fettsäure und/oder Fettsäuresalz in einer Konzentration von 15 bis 50 Gew.-% umfasst.

7. Körperpflegezusammensetzung nach einem vorhergehenden Anspruch, wobei das Fettacylisethionat-Produkt mehr als 20 Gew.-% und weniger als 45 Gew.-%, bevorzugter mehr als 25 Gew.-% und weniger als 45 Gew.-% der Kettenlänge von mehr als oder gleich C₁₆ umfasst und mehr als 50 Gew.-%, vorzugsweise mehr als 60 Gew.-% der freien Fettsäure/Seife eine Kettenlänge von C₁₆ bis C₂₀ aufweist.

8. Körperpflegezusammensetzung nach einem vorhergehenden Anspruch, wobei die Zusammensetzung eine Körperwaschzusammensetzung darstellt und die Tensidkombination 5 bis 60 Gew.-% umfasst.

9. Körperpflegezusammensetzung nach einem der Ansprüche 1-7, wobei die Zusammensetzung eine kosmetische Zusammensetzung ist und 1 bis 30 Gew.-% der Tensidkombination umfasst.

## Revendications

1. Composition pour le soin de la personne comprenant :
a) un tensioactif comprenant (i) un tensioactif anionique synthétique ; et (ii) un biotensioactif comprenant un glycolipide où le biotensioactif est présent à une teneur de 50 % en masse à 75 % en masse de la combinaison totale de tensioactif ; et
b) un produit d'iséthionate d'acyle gras à une teneur de 4 % en masse à 5 % en masse de la composition totale pour le soin de la personne.

2. Composition pour le soin de la personne selon l'une quelconque des revendications précédentes, où le biotensioactif comprend un rhamnolipide ou sophorolipide ou tréhalolipide ou un lipide de mannosylérythritol (MEL) ou des combinaisons de ceux-ci.

3. Composition pour le soin de la personne selon l'une quelconque des revendications précédentes, où le biotensioactif comprend un rhamnolipide.

4. Composition pour le soin de la personne selon l'une quelconque des revendications précédentes, où le biotensioactif comprend un sophorolipide

5. Composition pour le soin de la personne selon l'une quelconque des revendications précédentes, où le biotensioactif comprend une ou plusieurs moitiés saccharide.

6. Composition pour le soin de la personne selon l'une quelconque des revendications précédentes, où le produit d'iséthionate d'acyle gras comprend de l'iséthionate d'acyle gras à une teneur de 40 à 80 % en masse du produit, ainsi qu'un acyle gras et/ou sel d'acyle gras libre à une teneur de 15 à 50 % en masse.

7. Composition pour le soin de la personne selon l'une quelconque des revendications précédentes, où le produit d'iséthionate d'acyle gras comprend plus de 20 % en masse et moins de 45 % en masse, encore mieux plus de 25 % en masse et moins de 45 % en masse de longueur de chaîne supérieure ou égale à C₁₆ ; et plus de 50 % en masse, de préférence plus de 60 % en masse de l'acide gras libre/savon est de longueur de chaîne C₁₆ à C₂₀.

8. Composition pour le soin de la personne selon l'une quelconque des revendications précédentes, où la composition est une composition de lavage pour la personne et la combinaison de tensioactif comprend de 5 à 60 % en masse.

9. Composition pour le soin de la personne selon l'une quelconque des revendications 1-7 où la composition est une composition cosmétique et comprend de 1 à 30 % en masse de combinaison de tensioactif.
